# FASCICULE DE BREVET EUROPEEN

(11) **EP 1 150 632 B1**
(45) Date de publication et mention de la délivrance du brevet: **13.11.2002**
(21) Numéro de dépôt: 00902711.1
(22) Date de dépôt: 07.02.2000
(51) Int. Cl.: A61F 9/013

(54) **APPAREIL CHIRURGICAL POUR REALISER UNE DECOUPE LAMELLAIRE DE LA CORNEE**
CHIRURGISCHE VORRICHTUNG ZUR DURCHFÜHRUNG EINES LAMELLÄREN HORNHAUTSCHNITTES
SURGICAL DEVICE FOR LAMELLAR CUTTING OF THE CORNEA

(30) Priorité: 09.02.1999 FR 9901492
(43) Date de publication de la demande: 07.11.2001
(73) Titulaire: HumanOptics AG, 91054 Erlangen (DE)
(72) Inventeur: Hanna, Khalil, 75007 Paris (FR)
(74) Mandataire: Robert, Jean-Pierre
(86) Numéro de dépôt international: FR0000276
(87) Numéro de publication internationale: WO00047146

(56) Documents cités:
- EP-A- 0 895 765
- WO-A-94/06356
- WO-A-95/31143
- WO-A-98/10716
- WO-A-98/18517
- WO-A-98/27901
- WO-A-99/03433
- US-A- 4 173 980

## Description

La chirurgie corrective de l'amétropie selon la technique du Professeur BARRAQUER est maintenant, combinée au laser Excimer, une chirurgie développée et employée à grande échelle. Elle met en oeuvre des appareils chirurgicaux appelés microkératomes, comme, par exemple, celui décrit dans la publication internationale WO-A-98 27901, qui comportent essentiellement un anneau de fixation de l'oeil à opérer au centre duquel fait saillie la cornée et un appareil mobile de découpe guidé par rapport à l'anneau de fixation et qui comporte d'une part une surface aplanatrice de la partie de cornée faisant saillie à l'intérieur de l'anneau et d'autre part, une lame de découpe en général oscillante, dont le fil coupant est situé juste derrière la surface aplanatrice et à une distance déterminée de celle-ci mesurée verticalement de manière à définir l'épaisseur de la lamelle de cornée qui est ainsi soumise à résection.

La présente invention est une variante de réalisation des appareils connus de découpe de cornée mise en oeuvre dans la technique dite de BARRAQUER.

A cet effet elle concerne un appareil chirurgical pour réaliser une découpe lamellaire de la cornée comportant :
- une base en forme d'anneau conformée pour être appliquée et maintenue sur l'oeil,
- un outil de coupe comportant une lame de coupe logée dans un support coopérant avec la base pour guider un déplacement du fil de la lame dans un plan parallèle à l'anneau et comportant en avant de la lame un élément de conformation de la partie de cornée faisant saillie à l'intérieur de l'anneau, caractérisé en ce que l'élément de conformation est constitué par un rouleau attelé au support de lame autour d'un axe de rotation perpendiculaire à la direction de la coupe.

Ainsi, contrairement aux dispositifs existants, la conformation de la cornée n'est plus réalisée par un plateau qui écrase celle-ci mais par un organe qui roule sur la cornée sans glisser grâce auquel il est possible, d'une part d'obtenir une résection d'épaisseur uniforme ou non-uniforme dans le sens transversal, et d'autre part de faire varier l'épaisseur de cette résection au fur et à mesure de l'avancement de la découpe. Alors que le plateau aplanateur ne permet une résection que parallèle à la surface antérieure de la cornée, le rouleau de l'invention permet une résection ayant un profil variable et notamment un profil parallèle à la surface postérieure de la cornée dont on sait que l'épaisseur est plus importante en périphérie qu'au centre. On peut ainsi en une seule résection, modifier la courbure de la cornée pour corriger l'amétropie, après avoir déterminé quelle est la forme que doit adopter la surface extérieure de la cornée, donc le profil de cette dernière après correction. Il est également possible avec l'appareil selon l'invention de combiner une résection mécanique d'un volet de cornée de profil déterminé et ensuite d'y ajouter la résection supplémentaire d'un lenticule ou d'une autre partie du stroma avant de rabattre le volet.

Ainsi, dans l'appareil selon l'invention, le rouleau peut-il être soit cylindrique, soit en forme de tonneau, soit en forme de diabolo. Il peut également affecter toute forme utile et notamment être cylindrique et posséder une lentille rapportée en relief sur sa surface extérieure. De la même manière il peut être cylindrique et posséder un creux en forme de lentille sur sa surface extérieure. Dans le cas où sa surface extérieure n'est pas de révolution, le rouleau comporte un index lié à la position d'une génératrice particulière de sa surface périphérique et le support comporte un repère pour localiser cet index dans une position déterminée, notamment au début de l'opération.

De manière avantageuse, la surface périphérique de l'organe de conformation est pourvue de micro-reliefs pour favoriser son roulement sans glissement sur la surface de la cornée.

Par ailleurs et notamment pour les découpes de grand diamètre, il peut être utile de placer à l'avant du rouleau un plateau permettant d'éviter les variations de pression dans l'oeil. La distance entre la lame et le bord arrière du plateau est de l'ordre de 5 à 6 mm.

Enfin de manière également avantageuse, la base annulaire de maintien de l'oeil comporte sur sa face tournée vers l'oeil, une rainure cloisonnée par une paroi intermédiaire pourvue au moins d'une ouverture de communication des deux parties de la rainure que cette paroi détermine.

D'autres caractéristiques et avantages de l'invention ressortiront de la description de quelques variantes de réalisation de l'invention données ci-après à titre d'exemples non limitatifs.

Il sera fait référence aux dessins annexés parmi lesquels :
- la figure 1 est une vue schématique des moyens formant l'appareil selon l'invention,
- la figure 2 illustre quatre variantes de réalisation possibles d'un rouleau de conformation de la cornée,
- la figure 3 est une vue en coupe de l'anneau de maintien de l'oeil appartenant à l'appareil selon l'invention.

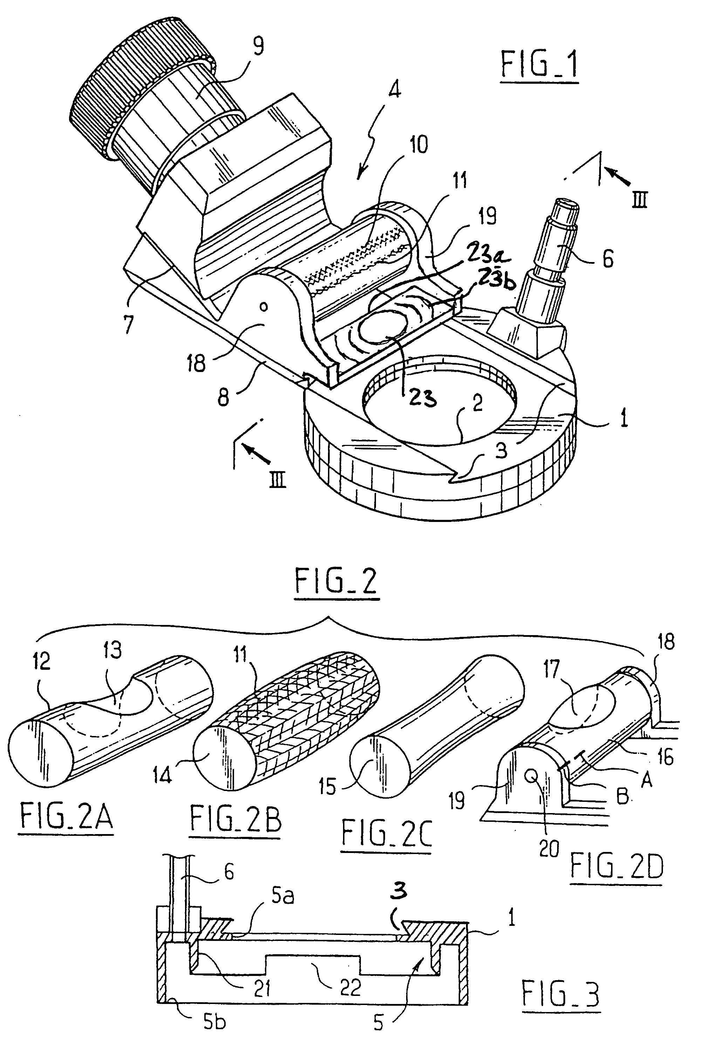

A la figure 1 on a représenté un anneau 1 de fixation d'un oeil à opérer qui est connu en lui-même et qui possède une ouverture 2 centrale au travers de laquelle la cornée fait saillie. Cet anneau est pourvu d'un guide 3, ici par exemple en forme d'une rainure en queue d'aronde dans lequel peut coulisser un chariot 4 constituant un porte-lame de découpe de la cornée. L'anneau 1 définit sur sa face en contact avec l'oeil (figure 3), un espace 5 limité par le bord inférieur 5b d'une jupe latérale et par l'arête inférieure 5a de l'ouverture 2 qui, lorsque ces deux lignes 5a et 5b sont en contact avec la conjonctive de l'oeil, définit une chambre qui peut être mise sous vide partiel au moyen d'un conduit 6. L'anneau 1 est ainsi fixé sur l'oeil par aspiration.

Le chariot 4 porte-lame représenté à la figure 1 n'a aucun caractère limitatif en ce qui concerne les moyens de l'invention. Aussi, l'invention s'applique-t-elle à tout kératome manuel ou automatique, c'est-à-dire motorisé, qui comporte un chariot porte-lame susceptible d'être déplacé dans un plan parallèle au plan de l'anneau 1 selon un trajet rectiligne ou courbe, grâce auquel il est possible de découper soit un volet soit un disque cornéen.

Le chariot 4 représenté comporte un logement 7 dans lequel, de manière connue, est placée une lame susceptible d'osciller transversalement au guide en queue d'aronde 8 que comporte ce chariot en partie inférieure, guide qui coopère avec la rainure en queue d'aronde 3 de l'anneau. Un élément 9 appartenant au chariot 4 veut symboliser les moyens de motorisation de la lame et/ou les moyens de motorisation du déplacement du chariot 4 par rapport à l'anneau 1.

En avant du fil de la lame situé en partie inférieure du logement 7, entre les guides 8, le chariot 4 porte un organe de conformation de la cornée formé par un rouleau 10. Ce rouleau est disposé de manière précise au-dessus du fil de la lame de manière que la partie de cornée déformée par ce rouleau ait une épaisseur contrôlée qui sera un critère déterminant de la résection réalisée. Le rouleau 10 est prévu pour rouler sans glisser sur la cornée et comporte à cet effet des micro-reliefs 11 sur sa surface extérieure.

La figure 2 illustre les variantes de réalisation du rouleau 10 permettant d'obtenir des profils de découpe particuliers. A la partie 2A de cette figure 2 on a représenté un rouleau 12 cylindrique duquel on a retiré un lenticule surfacique de sorte que ce rouleau possède un creux 13. A la partie 2B de cette figure, le rouleau 14 est en forme de tonneau. A la partie 2C, le rouleau 15 est quant à lui en forme de diabolo et à la partie 2d de la figure 2, le rouleau 16 est cylindrique avec une protubérance en forme de lentille 17 sur sa surface périphérique. La protubérance 17 peut être rapportée par tout moyen de fixation et. notamment par collage. La lentille 17 sera d'un diamètre adapté à l'intervention. Pour par exemple réaliser une résection parallèle à la face postérieure de la cornée, on utilisera une lentille à bord fin avec une épaisseur au centre supérieur de un à deux dixièmes de millimètres à l'épaisseur au bord selon le diamètre choisi.

Le support de rotation de chacun de ces rouleaux 10 à 16 est formé par deux bras prolongeant le chariot 4 vers l'avant-bras 18, 19, entre lesquels les rouleaux peuvent être encliquetés et assujettis fermement. Par exemple, les bras 18 et 19 peuvent recevoir un axe 20 qui serait monté non tournant dans ses bras 18 et 19 tandis que les rouleaux seraient formés par un organe tubulaire monté à roulement à billes sur l'axe fixe 20. Il convient en effet de réduire au maximum les frottements entre le rouleau et le chariot porte-lame 4 de manière que ce rouleau puisse rouler sans glisser sur la surface de la cornée.

On aura précisé que l'axe 20 du conformateur cylindrique de cornée selon l'invention est parallèle au fil de la lame de coupe, c'est-à-dire perpendiculaire au guide 8. En d'autres termes, cet axe est transversal par rapport au mouvement d'avance du chariot 4 dans la progression de la découpe de la cornée.

Enfin, on notera sur la partie 2D de la figure 2, la présence d'un index A sur le rouleau 16 qui peut être mise en face d'un repère B porté par l'un des bras 18, 19 du chariot 4, l'état de l'appareil lorsque l'index est en face du repère correspondant par exemple au démarrage d'une intervention. On comprend en effet que ces index et repère ne sont pas nécessaires si le rouleau est d'une surface extérieure de révolution autour de l'axe 20 ; ils le sont lorsque le profil de la découpe à obtenir est particulier et demande l'usage d'un rouleau de conformation qui a une surface extérieure n'étant pas de révolution autour de l'axe de rotation de ce rouleau.

On notera qu'à la figure 1 on a représenté entre les bras 18 et 19, à l'avant du rouleau 10, un plateau fixe dont la fonction est, en engendrant une précompression de la cornée, d'éviter les effets sur la découpe de la variation de pression dans l'oeil. Ce plateau est utile en particulier pour les découpes de grand diamètre. De manière préférée la distance séparant le bord arrière 23a du plateau de la lame de coupe est de 5 à 6 mm. En outre, ce plateau 23 sera avantageusement transparent avec un marquage 23b de contrôle dimensionnel de la portion aplanie de la cornée.

La figure 3 est une coupe diamétrale selon le plan III-III de la figure 1 de l'anneau 1. La section de cet anneau 1 est connue en elle-même, sauf en ce qui concerne la cloison 21 qui s'étend entre les bords 5a et 5b de la rainure 5 et qui forme chambre d'aspiration lorsque l'anneau est placé sur l'oeil, cette cloison étant destinée à reposer également sur la surface extérieure de l'oeil (la conjonctive) et ayant pour fonction d'éviter que, lors d'un arrachement ou d'un décollement de cette conjonctive, le débouché du tuyau 6 dans la rainure 5 ne se bouche. Afin de faire communiquer les deux parties de la rainure 5 ainsi cloisonnée, la paroi intermédiaire 21 comporte des embrèvements 22 qui assurent la communication permanente des deux parties.

## Revendications

1. Appareil chirurgical pour réaliser une découpe lamellaire de la cornée comportant :
- une base en forme d'anneau (1) conformée pour être appliquée et maintenue sur l'oeil,
- un outil de coupe comportant une lame de coupe logée dans un support (4) coopérant avec la base (1) pour guider un déplacement du fil de la lame dans un plan parallèle à l'anneau et comportant en avant de la lame un élément de conformation (10 ) la partie de cornée faisant saillie à l'intérieur de l'anneau,
**caractérisé en ce que** l'élément de conformation est constitué par un rouleau (10) attelé au support de lame autour d'un axe de rotation perpendiculaire à la direction de la coupe.

2. Appareil selon la revendication 1, **caractérisé en ce que** le rouleau est cylindrique.

3. Appareil selon la revendication 1, **caractérisé en ce que** le rouleau est en forme de tonneau.

4. Appareil selon la revendication 1, **caractérisé en ce que** le rouleau est en forme de diabolo.

5. Appareil selon l'une des revendications précédentes, **caractérisé en ce que** le rouleau (16) est cylindrique et possède une lentille (17) rapportée en relief sur sa surface extérieure.

6. Appareil selon l'une des revendications 1 à 4, **caractérisé en ce que** le rouleau (12) est cylindrique et possède un creux (13) en forme de lentille sur sa surface extérieure.

7. Appareil selon la revendication 5 ou la revendication 6, **caractérisé en ce que** le rouleau comporte un index A lié à la position d'une génératrice particulière de sa surface périphérique, et **en ce que** le support 4 comporte un repère B pour localiser l'index dans une position déterminée.

8. Appareil selon l'une des revendications précédentes, **caractérisé en ce que** la surface périphérique de l'organe de conformation est pourvue de micro-reliefs (11) pour favoriser son roulement sans glissement sur la surface de la cornée.

9. Appareil selon l'une quelconque des revendications, **caractérisé en ce qu'**il comporte, à l'avant du rouleau (10) un plateau (23) de précompression de la cornée.

10. Appareil selon la revendication 9, **caractérisé en ce que** le plateau (23) est transparent et comporte un marquage formant gabarit de contrôle de la cornée.

11. Appareil selon l'une des revendications précédentes, **caractérisé en ce que** la base annulaire (1) comporte sur sa face tournée vers l'oeil, une rainure (5) cloisonnée par une paroi intermédiaire (21) pourvue d'au moins une ouverture (22) de communication des deux parties de la rainure (5).

## Patentansprüche

1. Chirurgisches Gerät zum Durchführen eines lamellaren Hornhautschnittes, umfassend
eine ringförmige Basis (1), die ausgebildet ist, um auf das Auge aufgesetzt und dort gehalten zu werden,
ein Schneidewerkzeug mit einer Schneideklinge, die in einem Träger (4) angeordnet ist, der mit der Basis (1) zusammenwirkt, um eine Bewegung der Schneide der Klinge in einer zum Ring parallelen Ebene zu führen und der vor der Klinge ein Formelement (10) zum Formen des in das Innere des Ringes vorspringenden Abschnittes der Hornhaut trägt,
**dadurch gekennzeichnet, daß** das Formelement von einer Walze (10) gebildet ist, die an dem Träger der Klinge um eine zur Schneiderichtung senkrechte Drehachse drehbar gelagert ist.

2. Gerät nach Anspruch 1, **dadurch gekennzeichnet, daß** die Walze zylindrisch ist.

3. Gerät nach Anspruch 1, **dadurch gekennzeichnet, daß** die Walze tonnenförmig ist.

4. Gerät nach Anspruch 1, **dadurch gekennzeichnet, daß** die Walze die Form eines Diabolos hat.

5. Gerät nach einem der vorhergehenden Ansprüche, **dadurch gekennzeichnet, daß** die Walze (16) zylindrisch ist und eine auf ihrer Außenumfangsfläche reliefartig aufgetragene Linse (17) hat.

6. Gerät nach einem der Ansprüche 1 bis 4, **dadurch gekennzeichnet, daß** die Walze (12) zylindrisch ist und eine linsenförmige Vertiefung (13) in ihrer Außenfläche hat.

7. Gerät nach Anspruch 5 oder 6, **dadurch gekennzeichnet, daß** die Walze an der Position einer speziellen Erzeugenden ihrer Umfangsfläche einen Index (A) trägt und daß der Träger (4) eine Markierung (B) hat, um den Index in einer vorgegebenen Position zu lokalisieren.

8. Gerät nach einem der vorhergehenden Ansprüche, **dadurch gekennzeichnet, daß** die Umfangsfläche des Formelementes mit einem Mikrorelief (11) versehen ist, um sein gleitfreies Abrollen auf der Oberfläche der Hornhaut zu begünstigen.

9. Gerät nach irgendeinem der Ansprüche, **dadurch gekennzeichnet, daß** es vor der Walze (10) eine Platte (23) zum Vorkomprimieren der Hornhaut hat.

10. Gerät nach Anspruch 9, **dadurch gekennzeichnet, daß** die Platte (23) transparent ist und eine Markierung trägt, welche eine Kontroll-Lehre für die Hornhaut bildet.

11. Gerät nach einem der vorhergehenden Ansprüche, **dadurch gekennzeichnet, daß** die ringförmige Basis (1) an ihrer dem Auge zugewandten Seite eine Aussparung (5) hat, die durch eine Zwischenwand (21) unterteilt ist, die mit mindestens einer Verbindungsöffnung (22) zum Verbinden der beiden Abschnitte der Aussparung (5) versehen ist.

## Claims

1. A surgical instrument for lamellal cutting of the cornea, the instrument comprising:
• a ring-shaped base (1) shaped for being applied to and held against the eye; and
• a cutting tool having a cutting blade received in a support (4) co-operating with the base (1) to guide displacement of the cutting edge of the blade in a plane parallel to the ring, and including, in front of the blade, a shaping element (10) for shaping the portion of the cornea that projects into the ring;
the instrument being **characterized in that** the shaping element is constituted by a roller (10) coupled to the blade support about an axis of rotation perpendicular to the cutting direction.

2. An instrument according to claim 1, **characterized in that** the roller is cylindrical.

3. An instrument according to claim 1, **characterized in that** the roller is barrel-shaped.

4. An instrument according to claim 1, **characterized in that** the roller is diabolo-shaped.

5. An instrument according to any preceding claim, **characterized in that** the roller (16) is cylindrical and possesses a lens (17) fitted to project from its outer surface.

6. An instrument according to any one of claims 1 to 4, **characterized in that** the roller (12) is cylindrical and possesses a lens-shaped recess (13) in its outer surface.

7. An instrument according to claim 5 or claim 6, **characterized in that** the roller includes an index A associated with the position of a particular generator line of its peripheral surface, and **in that** the support 4 includes a mark B for locating the index in a determined position.

8. An instrument according to any preceding claim, **characterized in that** the peripheral surface of the shaper member is provided with microrelief (11) to enhance rolling without slip on the surface of the cornea.

9. An instrument according to any preceding claim, **characterized in that** it includes a cornea-pre-compressing plate (23) in front of the roller (10).

10. An instrument according to claim 9, **characterized in that** the plate (23) is transparent and includes graduations forming a template for inspecting the cornea.

11. An instrument according to any preceding claim, **characterized in that** the annular base (1) has a groove (5) in its face directed towards the eye, the groove (5) being partitioned by an intermediate wall (21) provided with at least one opening (22) providing communication between the two portions of the groove (5).
